Europäisches Patentamt

(19) European Patent Office

Office européen des brevets

(11) Publication number: **0 051 569**
**B1**

(12) **EUROPEAN PATENT SPECIFICATION**

(45) Date of publication of patent specification: **04.09.85**

(21) Application number: **81830205.1**

(22) Date of filing: **23.10.81**

(51) Int. Cl.⁴: **A 61 L 2/12**

(54) **A sterilisation device for surgical implements and instruments.**

(30) Priority: **04.11.80 IT 356480**

(43) Date of publication of application:
**12.05.82 Bulletin 82/19**

(45) Publication of the grant of the patent:
**04.09.85 Bulletin 85/36**

(84) Designated Contracting States:
**AT DE FR GB**

(56) References cited:
**CH-A- 293 571**
**DE-A-1 938 111**
**GB-A- 597 832**

(73) Proprietor: **ELETTRONICA LIARRE S.r.l.**
**Via Serraglio, 52**
**I-40026 Imola (Bologna) (IT)**

(72) Inventor: **Zambrini, Gianni**
**Via Serraglio, 54**
**I-40026 Imola (Bologna) (IT)**

(74) Representative: **Lanzoni, Luciano**
**c/o BUGNION S.p.A. Via Farini, 37**
**I-40124 Bologna (IT)**

EP 0 051 569 B1

Courier Press, Leamington Spa, England.

## Description

It has become the norm to refer to electromagnetic waves of frequencies ranging between hundreds of MHz and hundreds of GHz,—as microwaves. As a rule, microwave generation calls for particular kinds of electronic tube such as Clistron, Magnetron—in fact those which produce progressive-regressive waves.

Recently there has been seen a marked development in heating technology, with microwave generation being exploited in the production of kilns, drying ovens etc.,—more especially in the field of industry.

The object of the invention described herein is to provide a device for sterilising instruments used in surgery,—one which provides rapid and complete sterilisation by means of microwaves. This object is attained by the device to which the invention refers, which is characterised by the fact that it comprises: a microwave generator capable of producing frequencies equal to or greater than 2.45 GHz; a waveguide furnished with a contoured port from which microwaves exit; a sterilisation chamber bounded by totally reflective perimeter walls base and ceiling, into which chamber the said waveguide's exit port is located; at least one bearer element for the said instruments positioned inside the sterilisation chamber and positioned between the base and the ceiling and opposite thereto, the said bearer element being non-reflective to the said microwaves.

The appropriate contour given to the exit port of the abovementioned waveguide, plus the special structural shape of the sterilisation chamber and the adoption of bearer elements transparent to microwaves, combine to allow a thorough sterilisation of any kind of instrument in the shortest possible time and with maximum effect.

Further features and advantages of the invention will emerge more clearly from the detailed description of the device which follows, illustrated as a strictly unlimited example with the aid of the accompanying drawings, in which:—

Fig. 1 shows a view of the device described herein, in perspective, and with certain parts omitted the better to illustrate others;

Fig. 2 shows a longitudinal, vertical cross-section of a first form of embodiment of the waveguide in Fig. 1;

Fig. 3 shows a longitudinal, vertical cross-section of a second form of embodiment of the waveguide in Fig. 1;

Fig. 4 shows the same longitudinal vertical cross-section of the waveguide in Fig. 1—demonstrating a further possible variation on the embodiments illustrated in Figs. 2 & 3;

Fig. 5 shows a part cross-section of the waveguide exit port in order to demonstrate the orientation of microwaves therefrom;

Fig. 6 shows a schematic block-diagram of the device to which the invention refers.

With reference to the drawings, the device described herein consists of a command module SC fed from the mains, whose basic component is a Magnetron M generating microwaves of a frequency equal to or greater than 2.45 GHz which pass into a waveguide G from whence the latter distributes them through a contoured exit port B into a sterilisation chamber CS.

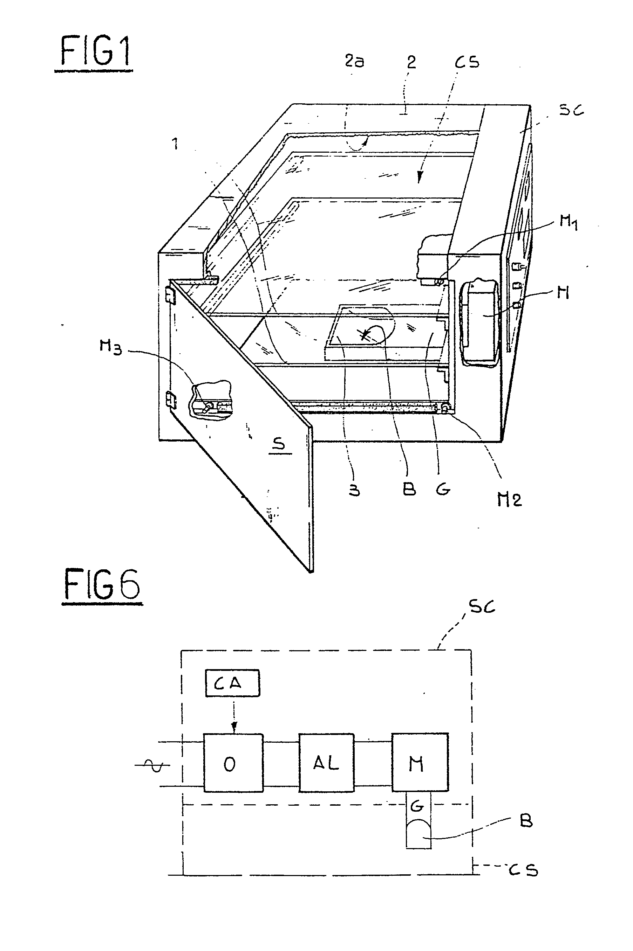

The command module SC (see Fig. 6) basically comprises a control unit O whose function is that of governing the duration of the device's use, and which is activated by signal derived from a safety circuit CA, the latter serving to ensure perfect closure of the door S by operating in conjunction with a group of at least three microswitches $M_1$ $M_2$ & $M_3$ (see Fig. 1). The command module also accommodates a feeder AL which serves the Magnetron M and which may be easily identified in Fig. 6. The sterilisation chamber CS—which in Fig. 1 is parallelepiped—is bounded by perimeter walls, base and ceiling which offer total reflection to microwaves. Provision is made therein for bearer elements 1, these being fashioned from a substance non-reflective to said microwaves, such as glass, Teflon®, or nylon. These elements 1 serve as surfaces upon which to deposit the said surgical instruments—thus exposing them on all sides to the microwaves emanating from exit port B. In order that the widest possible diversification of the microwaves' angle of incidence be achieved after leaving exit port B, at least one of the sterilisation chamber CS walls, for instance wall 2 as illustrated in Fig. 1 is provided with an undulating—rather than an even surface 2a. It will be obvious that the sterilisation chamber itself could be with one or more walls showing a curved surface area, or indeed be fashioned either cylindrically, or spherically, and so forth.

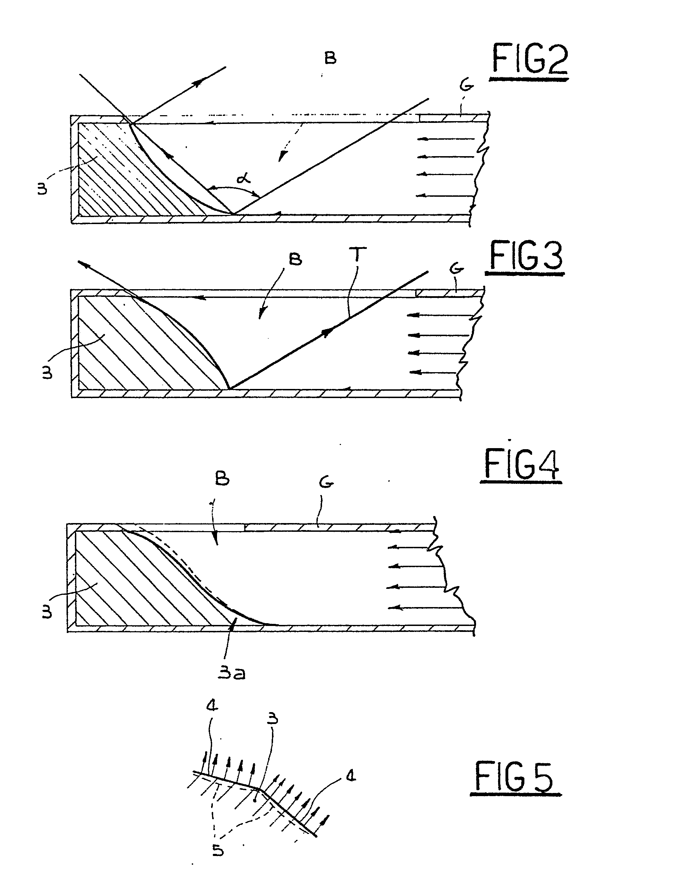

It is important, and should be underlined, that optimum microwave efficiency is obtained by beaming the waves in such a way that deviation is already produced at the exit port B of waveguide G. To this end, the said exit port B shows a sloping, or raked surface 3 for that precise purpose of beaming the microwaves multidirectionally on their exit from waveguide G. This said surface, or rake 3 may be produced either with a concave profile (Fig. 2) or a convex profile (Fig. 3), or again may be part-concave and part-convex or likewise vice versa as in Fig. 4. Clearly, any such curvature of said surface 3 will be such as to direct the microwaves out of exit port B along paths contained within a given angle "a"—the latter being sufficient for obtaining maximum efficiency of the device to which the invention refers.

In order to avoid "rebound" of microwaves back into waveguide G, it will suffice to ensure that the lower (or the uppermost) portion 3a of raked surface 3 is concave (or convex) as in Fig. 4. It is, in fact, in just this portion of the lower (or uppermost) extremity of raked surface 3 that such a problem might arise,—i.e. the possibility of microwaves rebounding back into the waveguide G.—viz. trajectory T produced by the convex rake 3 in Fig. 3.

The further possibility exists of combining a number of flat surfaces 4 to produce a rake 3, as in Fig. 5, these conjoined one with the next and offering differing angles with respect to the orientation of the microwaves—the latter being rectilinear and parallel with the longitudinal walls of waveguide G. With this arrangement groups of microwaves can be beamed so as to concentrate upon defined areas located in either a single, or a number of different planes.

A further form of embodiment could be one which seeks to assimilate the shape of a sine wave (see Fig. 5) of the type denoted by 5; this arrangement would enable pin-pointing of exact locations in the sterilisation chamber.

It will also be evident that the waveguide G itself may be made to assume a variety of configurations, all of which would ensure that microwaves emanating from exit port B be directed onto such paths as are necessary to the individual nature of the sterilisation being undertaken by the device thus described: in any way it is furnished with a microwave exit port shaped for total reflection of the microwaves and conformed in such a way that none of the microwaves are deflected back into the wave guide.

Finally, it should be noted that the internal surfaces of sterilisation chamber CS, as well as that raked surface 3 forming part of exit port B, will be of a 'specular' quality that is, offering total reflection to the microwaves thereby avoiding the least absorption of energy from the latter on the part of the same said surfaces.

**Claims**

1. A sterilisation device for surgical instruments characterised by the fact that it comprises: a microwave generator (M) producing frequencies equal to or greater than 2,45 G Hz; a waveguide (G) furnished with a contoured microwave exit port shaped for total reflection of the microwaves with no deflection back into the wave guide; a sterilisation chamber (CS) bounded by totally reflective perimeter walls, base and ceiling in which chamber the exit port (B) of said waveguide (G) is located; at least one bearer element (1) for said instruments, positioned within said sterilisation chamber (CS) between the base and the ceiling, said bearer element (1) being of a substance non-reflective to said microwaves.

2. Device according to claim 1 characterised by the fact that waveguide (G) incorporates a sloping, or raked surface (3) at the point of exit marked by said port (B).

3. Device according to claim 1 characterised by the fact that at least the wall (2) of said sterilisation chamber opposed to said exit port (B) is provided with an undulating rather than even surface.

4. Device according to claim 1 characterised by the fact that said sterilisation chamber (CS) has one or more walls with curved profile.

5. Device according to claims 1 or 2 characterised by the fact that said sloping or raked surface (3) is convex.

6. Device according to claims 1 or 2 characterised by the fact that said sloping or raked surface (3) is concave.

7. Device according to claims 1 or 2 characterised by the fact that said sloping or raked surface (3) is part-convex and part-concave.

8. Device according to claims 1, 2, 5, 6, 7 characterised by the fact that said sloping or raked surface (3) is composed of a number of flat surfaces (4) conjoined one with the next, and offering differing angles thus with respect to the orientation of microwaves emanating from said wave guide (G).

**Patentansprüche**

1. Einrichtung zum Sterilisieren chirurgischer Gerätschaften und Werkzeuge, dadurch gekennzeichnet, dass sie wie folgt enthält: Einen Mikrowellengenerator (M) mit einer Frequenz entsprechend oder stärker als 2,45 G Hz; eine Wellenführung (G), versehen mit einer profilierten Austrittsöffnung für die Mikrowellen, ausgebildet für eine totale Reflexion der Mikrowellen ohne Ableitung zurück in die Wellenführung; eine Sterilisationskammer (CS), gebildet aus vollkommen reflektierenden, umlaufenden Wänden, Boden und Decke, in der die Austrittsöffnung (B) der genannten Wellenführung (G) angeordnet ist; sowie wenigstens ein Ablageelement (1) für die genannten Gerätschaften, angeordnet in der genannten Sterilisationskammer (CS) zwischen dem Boden und der Decke, wobei das genannte Ablageelement (1) aus einem die genannten Mikrowellen nicht reflektierenden Material besteht.

2. Einrichtung nach Patentanspruch 1, dadurch gekennzeichnet, dass die Wellenführung (G) an dem Punkt, an dem sich die genannte Austrittsöffnung (B) befindet, eine schräge oder geneigte Oberfläche aufweist.

3. Einrichtung nach Patentanspruch 1, dadurch gekennzeichnet, dass wenigstens eine Wand (2) der genannten Sterilisationskammer, die der genannten Austrittsöffnung (B) gegenüberliegt, mit einer eher gewellten als ebenen Oberfläche versehen ist.

4. Einrichtung nach Patentanspruch 1, dadurch gekennzeichnet, dass die genannte Sterilisationskammer (CS) eine oder mehrere Wände mit einem gebogenen Profil aufweist.

5. Einrichtung nach den Patentansprüchen 1 oder 2, dadurch gekennzeichnet, dass die genannte schräge oder geneigte Fläche (3) konvex ausgebildet ist.

6. Einrichtung nach den Patentansprüchen 1 oder 2, dadurch gekennzeichnet, dass die genannte schräge oder geneigte Fläche (3) konkav ausgebildet ist.

7. Einrichtung nach den Patentansprüchen 1 oder 2, dadurch gekennzeichnet, dass die genannte schräge oder geneigte Fläche (3) teils konvex und teils konkav ausgebildet ist.

8. Einrichtung nach den Patentansprüchen 1, 2, 5, 6, 7, dadurch gekennzeichnet, dass die genannte schräge oder geneigte Fläche (3) aus einer Anzahl von flachen Oberflächen (4) besteht, die eine neben der anderen angeordnet und miteinander verbunden sind und folglich im Verhältnis zu dem Verlauf der aus der genannten Wellenführung (G) austretenden Mikrowellen unterschiedliche Winkelstellungen aufweisen.

**Revendications**

1. Dispositif de stérilisation pour du matériel et des instruments chirurgicaux, caractérisé en ce qu'il comporte: un générateur de micro-ondes (M) en mesure de produire des fréquences égales ou supérieures à 2,45 G Hz; und guide d'onde (G) pourvu d'un orifice de sortie des micro-ondes convenablement conformé pour la réflexion totale desdites micro-ondes sans aucune déflexion en arrière dans le guide d'onde; une chambre de stérilisation (CS) limitée par des parois périmétrales, une base et un plafond complètement réfléchissants, dans ladite chambre étant situé ledit orifice de sortie (B) du guide d'onde (G); au moins un élément de support (1) pour lesdits instruments, disposé à l'intérieur de la chambre de stérilisation (CS) et placé entre la base et le plafond, cet élément de support (1) étant obtenu de matière qui ne réfléchit pas lesdites micro-ondes.

2. Dispositif selon la revendication 1, caractérisé en ce que le guide d'onde (G) comporte une surface en pente ou inclinée (3) en correspondance du point de sortie indiqué par ledit orifice (B).

3. Dispositif selon la revendication 1, caractérisé en ce qu'au moins la paroi (2) de ladite chambre de stérilisation, en regard de l'orifice de sortie (B) est pourvue d'une surface ondulée au lieu de plate.

4. Dispositif selon la revendication 1, caractérisé en ce que ladite chambre de stérilisation (CS) est pourvue d'une ou de plusieurs parois à profil incurvé.

5. Dispositif selon les revendications 1 ou 2, caractérisé en ce que ladite surface en pente ou inclinée (3) est convexe.

6. Dispositif selon les revendications 1 ou 2, caractérisé en ce que ladite surface en pente ou inclinée (3) est concave.

7. Dispositif selon les revendications 1 ou 2, caractérisé en ce que ladite surface en pente ou inclinée (3) est partiellement convexe et partiellement concave.

8. Dispositif selon les revendications 1, 2, 5, 6, 7, caractérisé en ce que ladite surface en pente ou inclinée (3) se compose d'une pluralité de surfaces plates (4) unies l'une à l'autre et offrant des angles différenciés par rapport à l'orientation des micro-ondes émanant dudit guide d'onde (G).

FIG 1

FIG 6

FIG2

FIG3

FIG4

FIG5